(19) Europäisches Patentamt
European Patent Office
Office européen des brevets

(11) EP 1 480 601 B1

(12) FASCICULE DE BREVET EUROPEEN

(45) Date de publication et mention de la délivrance du brevet:
**11.04.2007 Bulletin 2007/15**

(21) Numéro de dépôt: **03735763.9**

(22) Date de dépôt: **28.02.2003**

(51) Int Cl.:
***A61Q 19/06*** *(2006.01)* ***A61K 8/68*** *(2006.01)*

(86) Numéro de dépôt international:
**PCT/FR2003/000656**

(87) Numéro de publication internationale:
**WO 2003/074011 (12.09.2003 Gazette 2003/37)**

(54) **UTILISATION COSMETIQUE DE LA PHYTOSPHINGOSINE COMME AGENT AMINCISSANT ET COMPOSITIONS COSMETIQUES CONTENANT DE LA PHYTOSPHINGOSINE**

KOSMETISCHE VERWENDUNG VON PHYTOSPHINGOSIN ALS SCHLANKMACHER UND PHYTOSPHINGOSIN ENTHALTENDE KOSMETISCHE ZUSAMMENSETZUNGEN

COSMETIC USE OF PHYTOSPHINGOSINE AS SLIMMING AGENT AND COSMETIC COMPOSITIONS COMPRISING PHYTOSPHINGOSINE

(84) Etats contractants désignés:
**AT BE BG CH CY CZ DE DK EE ES FI FR GB GR HU IE IT LI LU MC NL PT SE SI SK TR**

(30) Priorité: **01.03.2002 FR 0202675**

(43) Date de publication de la demande:
**01.12.2004 Bulletin 2004/49**

(73) Titulaire: **LVMH RECHERCHE**
**45800 St. Jean de Braye (FR)**

(72) Inventeurs:
- **FRANCHI, Jocelyne**
**F-45000 Orléans (FR)**
- **PELLICIER, Françoise**
**F-45470 Loury (FR)**

(74) Mandataire: **Giraud, Françoise et al**
**Cabinet Beau de Loménie**
**158, rue de l'Université**
**75340 Paris Cedex 07 (FR)**

(56) Documents cités:
**WO-A-95/03780** **US-A- 5 578 641**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 480 601 B1

Printed by Jouve, 75001 PARIS (FR)

## Description

Utilisation cosmétique de la phytosphingosine comme agent amincissant et compositions cosmétiques contenant de la phytosphingosine.

La présente invention concerne une nouvelle utilisation cosmétique de la phytosphingosine, comme agent amincissant ainsi que des compositions cosmétiques contenant de la phytosphingosine.

La phytosphingosine répond à la formule suivante :

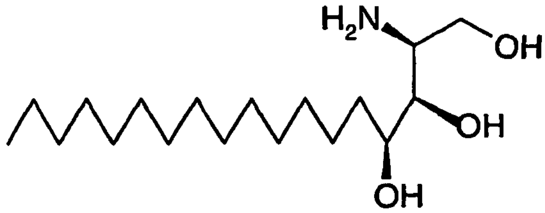

Sa formule moléculaire est $C_{18}H_{39}NO_3$ et son numéro CAS est le suivant : RN 100 000 403-19-8.

Ce produit est encore connu sous la dénomination : (2S,3S,4R)-2-amino-1,3,4-octadécanetriol.

La phytosphingosine est un produit commercial qui correspond à l'une des trois bases sphingoïde naturellement présentes dans la peau, la phytosphingosine étant présente dans le stratum corneum.

On connaît déjà des applications de la phytosphingosine et de ses sels et, plus particulièrement de son chlorhydrate, dans le domaine de la dermatologie. En effet, la phytosphingosine est essentiellement connue pour son activité antimicrobienne ainsi que pour son activité en tant que « second messager », application qui résulte en une réduction de la sensibilité de la peau. Plus précisément, la phytosphingosine est connue pour son activité dans le traitement de l'acné, pour son activité d'inhibiteur de la croissance des microorganismes sur la peau et pour réduire différents phénomènes inflammatoires observés sur la peau.

Les inventeurs de la présente invention ont maintenant découvert de façon tout à fait surprenante une nouvelle utilisation de la phytosphingosine ainsi que de ses sels cosmétiquement acceptables, en particulier de son chlorhydrate, comme agent amincissant. Ils ont par ailleurs mis en évidence que cette nouvelle utilisation était liée; au moins partiellement, à la propriété parfaitement inattendue de la phytosphingosine et de ses sels, de favoriser la synthèse de la leptine par les adipocytes de la peau.

Par ailleurs, poursuivant leurs études dans ce domaine, les inventeurs de la présente invention ont également mis en évidence qu'un certain nombre de combinaisons de la phytosphingosine ou de ses sels cosmétiquement acceptables s'avéraient particulièrement intéressantes dans ces nouvelles utilisations.

La leptine de la souris a été récemment identifiée en 1994 comme étant le produit du "gène ob" (Zhang, y et al., Nature, 1994, 372:425 et Tartaglia L.A., 1997, J. Biol. Chem. 272:6093).

La structure de la leptine humaine (ou protéine humaine OB) et son utilisation dans la modulation du poids chez les animaux ont été décrites dans la demande de brevet britannique GB 2292382. Il s'agit d'une protéine sécrétée par l'adipocyte qui informe le cerveau de l'état des réserves adipeuses. Elle agit par l'intermédiaire de récepteurs membranaires situés en particulier au niveau de l'hypothalamus.

D'abord étudiée chez le rongeur puis chez l'homme, elle joue un rôle clef dans la régulation du poids corporel.

Chez les souris ob/ob, l'absence de leptine dans le sérum due à des mutations du gène ob (celui qui code pour la leptine) entraîne une obésité massive.

Chez l'homme, les premiers travaux concernant la leptine ont été dirigés vers les patients obèses et/ou diabétiques.

En effet, plus un adipocyte possède une teneur élevée en triglycérides, plus il produit de leptine, et inversement (Médecine/Sciences, 1998, n°8-9, 14, 858-864, G. Ailhaud : L'adipocyte, cellule secrétrice et endocrine).

Ainsi chez les obèses, deux situations peuvent se présenter. Soit il existe une mutation du gène de la leptine, celle-ci est alors non fonctionnelle, en particulier sur les récepteurs du cerveau. Soit il existe un défaut du transfert de la leptine au niveau de la barrière entre circulation sanguine et cerveau.

Une étude, au cours de laquelle une injection quotidienne de leptine synthétique a été réalisée sur des patients qui souffraient d'obésité

ou de surcharges pondérales a montré des résultats probants : une perte de poids significative chez les patients atteints d'une certaine forme d'obésité est apparue (travaux menés à la Tufts University aux Etats-Unis, présentés à l'occasion du congrès American Diabetes Association : Jean Mayer, USA Human Nutrition Research Center on Aging).

En fait, la leptine déclenche un phénomène de satiété provoquant une diminution de la prise alimentaire et

diminuant la fréquence des prises alimentaires.

Lorsque la masse adipeuse augmente, la leptine produite par le tissu adipeux va inhiber la prise alimentaire et stimuler la dépense énergétique. Elle va donc s'opposer ainsi à une prise de poids excessive.

Ainsi donc, on peut considérer que cette protéine est un régulateur de la masse adipeuse dont le rôle primordial est d'inhiber le dépôt d'adiposité en excès.

Le rôle de régulation locale joué par la leptine est bien connu (voir Systemically and Topically Administered Leptin Both Accelerate Wound Healing in Diabetic ob/ob Mice. B.D. Ring, S. Scully, C.R. Davis, M.B Baker, MJ. Cullen, M.A. Pelleymounter, D. Danilenko. Endocrinology, 2000 vol. 141, n°1, p. 446-449).

Par ailleurs, le rôle de la leptine dans l'expression de certains gènes conduisant à l'accumulation de lipides (gènes de différenciation) est également bien connu dans le cadre de la régulation de la lipolyse.

La leptine, d'une part réprime l'expression de certains gènes conduisant à l'accumulation de lipides (gènes de différenciation), et cela sans la participation du cerveau.

D'autre part, la leptine induit une lipolyse directement sur les adipocytes. Ceci a été observé sur des adipocytes de souris in vitro (voir In vitro Lipolytic Effect of Leptin on Mouse Adipocytes : Evidence for a possible Autocrine/Paracrine Role of Leptin, G. Frühbeck, M. Aguado, J.A. Martinez, Biochemical and Biophysical Research communications 1997 : 240, p. 590-594). L'effet de la leptine sur la lipolyse des adipocytes est spécifique et opère via des récepteurs présents dans le tissu adipeux blanc.

La leptine transforme l'oestrone de la circulation sanguine (hormone qui augmente le dépôt de lipides) en oléyl-oestrone qui est considéré comme un facteur "amincissant". L'apparition de ce facteur provoque une lipolyse généralisée et une thermogénèse (voir Leptin enhances the synthesis of oleyl-estrone from estrone in white adipose tissue, M. Esteve, J. Virgili, H. Aguilar, F. Balada, J.A. Fernandez-Lopez, W. Remesar, M. Alemany, Eur J. Nutr. 1999, 38, p. 99-104). Administrée à des rats obèses ou normaux, l'oléyl-oestrone provoque une perte de masse grasse.

On voit donc tout l'intérêt qu'il y a à disposer d'un moyen pour agir sur la synthèse de la leptine qui agira notamment:

- directement au niveau cutané par l'intermédiaire des récepteurs présents à ce niveau,
- sur le tissu adipeux en provoquant la lipolyse,
- sur un facteur agissant comme régulateur du poids, à savoir l'oléyl-oestrone.

Des essais réalisés dans le cadre de la présente invention, aussi bien sur des cultures d'adipocytes murins que sur des cultures d'adipocytes humains ont permis de montrer qu'il était possible, par traitement de ces cultures avec de la phytosphingosine ou l'un de ses sels, en particulier son chlorhydrate, de stimuler la synthèse de la leptine par ces adipocytes, ce qui laissait présager la possibilité d'utiliser la phytosphingosine ou ses sels comme agent amincissant. Cet effet a pu être confirmé.

Ainsi donc, la présente invention concerne selon un premier aspect une nouvelle utilisation cosmétique de la phytosphingosine ou d'un de ses sels cosmétiquement acceptables, en particulier son chlorhydrate, en tant qu'agent amincissant.

Selon un deuxième aspect, l'invention concerne une nouvelle utilisation cosmétique de la phytosphingosine ou de l'un de ses sels cosmétiquement acceptables, en particulier de son chlorhydrate, comme agent actif stimulant la synthèse de la leptine par les adipocytes,

Selon un troisième aspect, l'invention concerne un procédé de traitement cosmétique destiné à obtenir un effet amincissant du corps humain, selon lequel on applique sur les parties du corps où ledit effet est recherché une quantité efficace d'une composition cosmétique contenant de la phytosphingosine ou l'un de ses sels cosmétiquement acceptables, en particulier son chlorhydrate.

Par ailleurs, il est apparu que, selon les trois aspects de l'invention tels qu'ils sont définis ci-dessus, certaines combinaisons de phytosphingosine ou d'un de ses sels cosmétiquement acceptables, en particulier de son chlorhydrate s'avéraient particulièrement intéressantes, pour améliorer l'effet amincissant obtenu par application de l'une quelconque des compositions contenant ces associations particulières.

Plus précisément, il est apparu que l'association de la phytosphingosine ou d'un de ses sels cosmétiquement acceptables, avec un ou plusieurs agents, ci-après désignés par agents lipolytiques, induisant une lipolyse, dans les adipocytes s'avérait particulièrement intéressante dans le cadre de la présente invention, comme cela sera expliqué plus loin.

En particulier, on choisira pour réaliser cette association au moins un agent lipolytique cosmétiquement acceptable dans le groupe constitué par l'adénosine-3',5'-cyclique monophosphate (AMPc) et ses dérivés, les agents activateurs de l'enzyme adénylate cyclase et les agents inhibiteurs de l'enzyme phosphodiestérase.

Comme agent activateur de l'adénylate cyclase, on choisira avantageusement la forskoline ou un extrait de plante en contenant, tel qu'un extrait de *Coleus forskohlii* ou *Plectranthus barbatus,* ou encore un extrait de la plante *Tephrosia purpurea.*

Comme agent inhibiteur de phosphodiestérase, on pourra utiliser une xanthine telle que la 3-isobutyl-1-méthyl-xanthine ou IBMX, la caféine ou la théophilline.

Les compositions cosmétiques renfermant de telles associations, constituent le quatrième aspect de la présente invention. Ce sont ces compositions telles qu'elles sont décrites ci-après qui seront de préférence mises en oeuvre dans toutes les applications cosmétiques visées par la présente invention.

Ainsi donc selon ce quatrième aspect, la présente invention concerne une composition cosmétique, notamment destinée à réduire les surcharges graisseuses sous-cutanées, caractérisée en ce qu'elle contient, à titre d'agent actif,

- de la phytosphingosine ou un de ses sels cosmétiquement acceptables, en particulier son chlorhydrate et
- au moins un agent lipolytique cosmétiquement acceptable choisi dans le groupe constitué par l'AMPc et ses dérivés cosmétiquement acceptables, les agents activateurs de l'enzyme adénylate cyclase et les agents inhibiteurs de l'enzyme phosphodiestérase,

dans un véhicule cosmétiquement acceptable.

Dans les compositions nouvelles de l'invention qui sont également les compositions préférées pour la mise en oeuvre des différentes applications visées par la présente invention, la phytosphingosine ou l'un de ses sels cosmétiquement acceptables, en particulier son chlorhydrate, est contenu dans la composition cosmétique à une concentration comprise entre 0,001 % et 1 % et, de préférence, entre 0,05 % et 0,5 % en poids par rapport au poids total de ladite composition.

La composition cosmétique contient en outre au moins un agent actif lipolytique choisi dans le groupe constitué par l'AMPc et ses dérivés lipolytiques, les agents activateurs de l'enzyme adénylate cyclase et les agents inhibiteurs de l'enzyme phosphodiestérase.

Dans ces compositions cosmétiques, l'AMPc ou son dérivé sera avantageusement utilisé à une concentration comprise entre à 0,001 % et 5 % en poids par rapport au poids total de la composition.

A titre de dérivé de l'AMPc, on pourra choisir tout dérivé de l'AMPc cosmétiquement acceptable et en particulier un sel ou un dérivé acylé, notamment un dérivé mono- ou dibutyryle.

A titre d'agent activateur de l'enzyme adénylate cyclase, on choisit, de façon avantageuse, la forskoline ou un extrait de plante en contenant, de préférence à une concentration comprise entre 0,001 % et 1 % et, de préférence entre 0,05 % et 0,25 %, en poids par rapport au poids total de la composition.

Comme extrait contenant de la forskoline, on choisira de préférence un extrait de *Coleus forskohlii* ou *Plectranthus barbatus.* Un tel extrait peut être obtenu par un procédé d'extraction tel que celui décrit dans la demande internationale WO 91/02516.

On pourra également utiliser comme agent activateur de l'adénylate cyclase un extrait de la plante *Tephrosia purpurea,* à une concentration comprise entre 0,001 % et 5 %, de préférence entre 0,01 % et 5 %, en poids par rapport au poids total de la composition. Un tel extrait peut être obtenu par un procédé d'extraction tel que celui décrit dans la demande internationale WO 95/03780.

Enfin, comme exposé précédemment, selon une autre variante, les compositions préférées selon l'invention contiennent un agent inhibiteur de la phosphodiestérase, en particulier une xanthine et, tout particulièrement, la 3-isobutyl-1-méthyl-xanthine (IBMX), la caféine ou la théophilline, de préférence à une concentration comprise entre 0,001 % et 10 %, de préférence entre 0,01 et 1 %, en poids par rapport au poids de la composition.

Les compositions préférées utilisées selon la présente invention et qui contiennent une association de phytosphingosine ou de l'un de ses sels avec un agent lipolytique tel que l'AMPc et ses dérivés lipolytiques, les agents activateurs de l'adénylate cyclase et les agents inhibiteurs de la phosphodiestérase, s'avèrent particulièrement intéressantes du fait de l'action synergique des deux types de constituants.

Sans que les inventeurs de la présente invention s'estiment totalement liés par cette explication, une interprétation plausible de l'effet de synergie observé est donnée ci-après.

Il a en effet été déjà observé que les agents favorisant une lipolyse dans les adipocytes, tels que les extraits de *Coleus* par exemple, présentent une efficacité biologique remarquable qui associe en général un important pouvoir lipolytique à une activité inhibitrice de la maturation adipocytaire. La réduction importante en volume et en quantité des vacuoles lipidiques après un traitement par l'agent lipolytique conduit à une réduction de la production de leptine. Il est donc probable que cette perte locale de leptine dans l'environnement proche des adipocytes résultant du traitement par l'agent lipolytique puisse être compensée par l'effet d'un produit stimulant la synthèse de la leptine, dans le cas présent par la phytosphingosine ou son sel. Le maintien d'une concentration suffisante en leptine dans l'environnement proche des adipocytes exerce ainsi un rôle qui s'oppose à l'augmentation de la masse adipeuse. Tout semble donc se passer comme si le message était émis par des cellules grasses qui informent par une opération de rétro-contrôle de la nécessité de réduire le stockage sous forme de triglycérides. Ainsi, grâce à l'action combinée de la phytosphingosine ou de l'un de ses sels, et d'au moins un autre agent lipolytique, on obtient un effet amincissant renforcé et plus prolongé.

Cette hypothèse semble tout à fait confirmée par les résultats obtenus dans le cadre des exemples 2 et 4 de

la présente invention portant sur l'association de la phytosphingosine avec un extrait de *Coleus forskohlii*.
Les exemples qui suivent sont donnés à titre purement illustratif de la présente invention. Ils sont accompagnés des figures 1 à 6, qui représentent respectivement :

- figure 1 : l'effet sur la lipogénèse de différents traitements réalisés à l'exemple 2 ;
- figures 2 à 4 : la morphologie des adipocytes murins à différentes étapes du traitement selon l'exemple 2 ;
- figure 5 : la morphologie des adipocytes humains à J18 pour une solution témoin (selon l'exemple 3) ; et
- figure 6 : la morphologie des adipocytes humains à J18 pour un traitement par une association selon l'invention (selon l'exemple 3).

**EXEMPLES**

Dans les exemples qui suivent et, sauf indications contraires, les proportions sont indiquées en pourcentage en poids.

Exemple 1

Mise en évidence de l'activité stimulante de la phytosphingosine sur la production de leptine par des adipocytes murins en culture.

1. Principe du test

Le concept selon lequel la masse adipeuse peut être régulée via des facteurs circulants sécrétés est très intéressant.
Le principe du test est de contrôler la sécrétion de la leptine par les adipocytes.

2. Matériel et méthodes

- Culture de cellules 3T3 F442A

A partir d'une lignée établie de préadipocytes de souris 3T3, a été isolé un clone qui a la capacité d'accumuler de grandes quantités de triglycérides. Les agents lipolytiques réduisent cette accumulation. Il est donc apparu pertinent de tester des agents lipolytiques potentiels sur cette lignée de préadipocytes murins 3T3 F442A. Ces préadipocytes (GREEN H. and KEHINDE O. - Spontaneous Heritable Changes Leading to Increased Adipose Conversion in 3T3 Cells, Cell Vol. 7, 105-113, 1976) peuvent se multiplier et se différencier en présentant le phénotype morphologique et biochimique caractéristique de la fonction différenciée de l'adipocyte mature. Lorsqu'ils sont en phase exponentielle de croissance, ils sont d'apparence fibroblastique, présentent une forme allongée et sont très adhérents au support. A confluence lorsque les conditions le permettent, une transition morphologique très précoce leur confère une forme arrondie.
Les cellules connaissent alors un processus d'amplification clonale. A ces changements morphologiques s'ajoutent des accroissements de l'activité d'enzymes lipogénétiques ainsi que des augmentations de réponses de la part des cellules à des hormones/facteurs affectant la lipogénèse et la lipolyse.
Les préadipocytes 3T3 F442A constituent donc un excellent modèle d'étude de la lipolyse du fait des transformations morphologiques et métaboliques acquises par les cellules pendant leur programme de développement. (Pairault J and Lasnier F : Control of adipogenetic différenciation of 3T3 F442A cells by retinoic acid, dexamethasone and insulin : a topographic analysis J. cell Physiol. 1987, 132, 279-86).
D'après la littérature, la leptine est sécrétée dans le milieu de culture puisqu'elle est stockée dans les adipocytes. (Wabitsch M et al, Diabetes, 1996, vol 45, Bornstein S. et al, Diabetes, 2000, vol 49, Friedman JM, Nutrition Reviews, 1998, vol 56 n°2).
Dans les cellules 3T3 F442A, l'expression du gène ob a été particulièrement étudié (Leroy P et al, J. of Biol. Chem, 1996, vol 271 n°5, pp. 2365-2368, Considine RV et al - Horm. Res. 1996, 46 : 249-256).
Les préadipocytes 3T3 F442A sont ensemencés à J0 dans les boites de Pétri 35 mm (Corning) et mis à l'étuve à 37°C sous une atmosphère air-$CO_2$ (95-5). Les cellules sont cultivées dans un milieu essentiel minimum de Eagle modifié selon Dulbecco (glucose 4,50 g/l) (DMEM-GIBCO BRL) complémenté avec 5 % de sérum de veau (SV) (BIOMEDIA®) et 5 % de sérum de veau foetal (GIBCO) pendant la phase de croissance. Le milieu est changé à J2 et J4.
A la confluence cellulaire (à J7), le milieu est changé :
Le milieu de base reste le même (DMEM) mais est supplémenté avec 10 % de sérum de veau foetal (SVF) et de l'insuline (5 μg/mL) (SIGMA).
Le milieu est ensuite changé à J9 et J11.

A J14, J16 et J18, on effectue un traitement par la composition dont on veut contrôler l'efficacité sur la synthèse de la leptine.

Le protocole est résumé dans le tableau ci-dessous :

| | |
|---|---|
| J0 | Ensemencement des 3T3 F442A dans DMEM, 5 % SV, 5 % SVF - densité cellulaire 2 x $10^4$ cellules / boîte de Pétri 35 mm |
| J2 | Changement de milieu |
| J4 | Changement de milieu |
| J7 | Confluence - Milieu de culture DMEM, 10 % SVF, 1 % insuline (solution mère à 500 $\mu$g/mL) |
| J9 et J11 | Changement de milieu |
| J14 | Traitement par la composition à tester |
| J16 et J18 | Traitement par la composition à tester |
| J21 | Collecte des surnageants cellulaires |

3. Dosage de la leptine

La leptine sécrétée est déterminée au moyen d'une technique Elisa de type sandwich en recourant au kit Quantikine® M Mouse Leptin Immunoassay.

Ce dosage ELISA utilise la leptine de souris recombinée, exprimée dans E.Coli et des anticorps dirigés contre la leptine recombinante de souris.

Le test utilise une technique immunoenzymatique "en sandwich". On tapisse les puits des microplaques avec un anticorps polyclonal de la leptine de souris.

Les standards, contrôles et échantillons sont déposés dans les puits et à ce moment là, toute la leptine présente se lie à l'anticorps immobilisé.

La leptine liée est ensuite détectée par un anticorps anti-leptine de souris couplé à une enzyme, la peroxydase. Une solution substrat est ensuite ajoutée dans les puits. La réaction enzymatique conduit à une solution bleue qui vire au jaune après ajout d'une solution d'arrêt.

L'intensité de la couleur mesurée est proportionnelle à la quantité de leptine présente. La lecture de la densité optique est faite à 450 nm au spectrophotomètre.

On obtient après le dosage des courbes doses-réponses concernant la mesure de la leptine naturelle, parallèle aux courbes standard obtenues avec les standards Quantikine M "recombinants". Le kit Quantikine M permet donc de déterminer des valeurs en masse relative pour la leptine de souris naturelle.

La densité optique mesurée à 450 nm est proportionnelle à la quantité d'anticorps fixé, elle-même proportionnelle à la quantité de leptine initialement présente. Les résultats sont exprimés en pg/mL de leptine présents dans les surnageants cellulaires.

Les échantillons ont été testés en triplicate.

4. Résultats

Trois compositions contenant respectivement 0,25, 1 et 2 $\mu$g/mL de phytosphingosine ou de son chlorhydrate ont été testées.

Pour les adipocytes 3T3-F442A maintenus en culture sans aucun traitement, la quantité de leptine présente dans les surnageants de culture, augmente fortement au cours du temps (16.4 pg/mL à J4 , 802 pg/mL à J11 et 2623 pg/mL à J20). Ce résultat est en conformité avec les données bibliographiques (Leroy P. 1996, Considine RV. 1996) : dans des conditions basales, les adipocytes murins sécrètent des quantités croissantes de leptine tout au long de leur maturation. Nous confirmons ainsi qu'un adipocyte mature sécrète des quantités de leptine supérieures à celles d'un préadipocyte.

Dans le tableau 1 ci-dessous sont reportés les quantités de leptine présentes dans les surnageants. L'abréviation PS désigne la phytosphingosine.

Tableau 1

| Quantité de leptine, exprimée en pg/mL présente dans les surnageants de culture d'adipocytes 3T3-F442A matures traités par la phytosphingosine au jour J21 | | |
|---|---|---|
| phytosphingosine (μg/mL) | leptine (pg/mL) | |
| | moyenne | écart |
| O (témoin) | 1510 | 23,43 |
| 0,25 | 1789 | 11,27 |
| 1 | 1563,6 | 14,33 |
| 2 | 1428,8 | 200 |

Après 7 jours de traitement, c'est à dire au jour J21, la phytosphingosine induit une augmentation de la sécrétion de leptine par les adipocytes traités, effet qui est maximal à la concentration de 0,25 μg/mL A cette concentration, l'augmentation est d'un peu plus de 18%.

Testé dans les mêmes conditions, le chlorhydrate de phytosphingosine est responsable également d'une stimulation de la sécrétion de leptine : + 52% à 1 μg/mL et + 26% à 2 μg/mL et + 18% à 0.25 μg/mL. Les résultats pour le chlorhydrate sont reportés au tableau 2 ci-dessous.

Tableau 2

| Quantité de leptine, exprimée en pg/mL présente dans les surnageants de culture d'adipocytes 3T3-F442A matures traités par le chlorhydrate de phytosphingosine au jour J21 | | |
|---|---|---|
| phytosphingosine- HCl (μg/mL) | leptine (pg/mL) | |
| | moyenne | écart |
| 0 (témoin) | 1510 | 23,43 |
| 0,25 | 1789 | 201,79 |
| 1 | 2301,3 | 49,821 |
| 2 | 1907,9 | 31,93 |

Ainsi, la phytosphingosine est capable d'induire dans la cellule adipeuse 3T3-F442A, modèle très proche de l'adipocyte humain, une augmentation de la sécrétion adipocytaire basale de leptine. La phytosphingosine est donc capable de jouer un rôle important dans le contrôle de la stabilité de la masse graisseuse.

Exemple 2

Mise en évidence de intérêt de l'association de la phytosphingosine avec un activateur d'adénylate cyclase, tel qu'un extrait de *Coleus forskohlii,* pour favoriser la diminution de la lipogénèse dans les adipocytes murins en culture.

1. Principe de l'étude

Cette étude concerne les effets de l'association des 2 actifs *Coleus forskohlii* (encore nommé *Plectranthus barbatus)* (PB) et Phytosphingosine (PS) sur le recrutement de nouveaux adipocytes.

Il est connu que le développement du tissu adipeux blanc représente un processus continu tout au long de la vie (AILHAUD G., GRIMALDI. P., NEGREL R., Trends in Endocrinology and Metabolism, (1994) 5 (3) 132-6). L'adipocyte est associé au sein du tissu adipeux , à une abondante matrice extracellulaire qui renferme également des cellules endothéliales, des capillaires, des fibres nerveuses et des fibroadipoblastes précurseurs. L'adipocyte mature représente le phénotype d'une cellule issue de la différenciation d'un précurseur adipocytaire. Les préadipocytes sont présents au sein même du tissu adipeux et peuvent être recrutés à tous les stades de la vie pour générer de nouveaux adipocytes : dans le cas d'un gain de poids, il existe une phase initiale d'augmentation de volume adipocytaire jusqu'à atteindre un point critique, conduisant ensuite au recrutement de nouvelles cellules ( Bjorntorp P., Int. J. Obesity, (1991) 15 67-81). La capacité intrinsèque des préadipocytes à se multiplier et à se différencier en adipocytes joue un rôle déterminant dans le développement des masses graisseuses. Une hyperplasie de ces cellules apparentées aux fibroblastes conduit

à une augmentation du tissu adipeux.

Ainsi, dans un premier temps, des adipocytes matures sont traités par PB + PS

Dans un deuxième temps, le milieu de culture conditionné par ces adipocytes est mis en contact avec des préadipocytes dont on va suivre la maturation en adipocytes.

Les cellules témoins au début du traitement, commencent à se différencier et subissent un certain nombre de changements : augmentation de volume, augmentation du nombre et de la taille des gouttelettes lipidiques, accroissement de l'activité d'enzymes lipogénétiques etc.

Une enzyme clé dans le processus de synthèse des triglycérides est la glycérol-3-phosphate deshydrogénase ($G_3PDH$): son activité spécifique augmente considérablement au cours de la maturation et peut donc être utilisée comme une mesure précise et sensible de la conversion adipocytaire (Pairault J., Green H., Proc. Nat. Acad. Sci. USA, (1979) 76, 5138-42; Koekemoer T.C.et al, Int. J. Biochem. Cell Biol. (1995) 27, 625-32).

On a choisi de suivre l'évolution de l'activité de cette enzyme pour mesurer l'état de différenciation des adipocytes.

La $G_3PDH$ étant une enzyme hydrosoluble, on mesure son activité dans le surnageant du broyat cellulaire en présence des substrats appropriés (NADH, TEA (triéthanolamine) - EDTA, 50 mM, 1 mM).

A partir des dosages, l'activité spécifique est calculée. On compare les cellules traitées aux cellules témoins. La $G_3PDH$ étant le reflet de l'état de différenciation des cellules, plus son activité spécifique est importante et plus les cellules sont différenciées et inversement : plus la réserve de graisse sera limitée par l'agent testé plus l'activité de la $G_3PDH$ sera faible.

La moyenne sur trois mesures rapportées à un écart type donne l'activité spécifique moyenne, ensuite on calcule le pourcentage d'inhibition de l'activité de la $G_3PDH$ produit par les substances comparé aux témoins.

Les critères permettant d'assurer la qualité de bons agents anti-lipogénétiques sont d'une part un pourcentage d'inhibition de l'enzyme supérieur à 50 % et d'autre part des données brutes significativement différentes par rapport aux témoins.

2. Matériel et méthodes

a) Culture et traitements

Les adipocytes en cours de différenciation, adipocytes peu matures encore dénommés préadipocytes obtenus à J7 du protocole donné dans l'exemple 1 sont traités par du milieu conditionné par des adipocytes différenciés matures traités ou non par PB ou PS et par l'association PB + PS pendant 8 jours avec un changement de milieu aux jours J9 et J11, comme indiqué dans le protocole ci-après.

La phytosphingosine a été testée aux concentrations finales de 0,25, 0,50 et 1 μg/mL.

L'extrait de *Plectranthus barbatus* (PB) (lot n° 0B2, INDENA) est titré à 80 % en forskoline, molécule reconnue pour être effectrice de l'adénylate cyclase (Seamon K. et al., P.N.A.S. USA, (1981) 78 3363-67). La concentration de PB utilisée dans l'étude est de 25 μg/mL à partir d'une solution mère à 20 mg/mL dans l'éthanol.

b) Préparation des extraits cellulaires

Le tapis cellulaire est lavé 2 fois avec du tampon PBS et les cellules sont récupérées par grattage dans du tampon 25 mM, TRIS - HCl, pH 7,5 contenant 1 mM d'EDTA à 4°C. Les cellules sont homogénéisées par broyage et centrifugation à 10 000 g pendant 10 minutes à 4°C.

Le protocole suivi est résumé dans le tableau ci-dessous.

| | |
|---|---|
| J0 | Ensemencement des 3T3F442A - densité cellulaire 2 x $10^4$ cellules / boîte de Pétri 35 mm. Milieu de culture DMEM, 5% SVF/5 % SV. |
| J2 | Changement de milieu |
| J4 | Changement de milieu |
| J7 | Confluence - Milieu de culture DMEM, 10 % SVF, 1 % insuline (SM 500 μg/mL) Traitement par les milieux conditionnés : PB : 0,25 μg/mL PS: 0,5 et 1 μg/mL |
| J9-J11 | Changement de milieu, même milieu + traitement |
| J14 | Traitement, même milieu de culture |
| J16 | Recueil des surnageants et dosage $G_3PDH$ |

c) Détermination de l'activité glycéro-3-phosphate déshydrogénase ($G_3PDH$)

Le dosage de l'activité $G_3PDH$ est effectué selon la méthode de Kozak et Jensen : Kozak et Jensen, 1974, J. Biol. Chem., 249, 7775-7781.

La $G_3PDH$ catalyse la réaction suivante:

$$\underset{\text{(DHAP)}}{\text{Dihydroxyacétone phosphate}} + \text{NADH}, H^+ \xrightarrow[G_3PDH]{} \text{glycérol 3 phosphate} + NAD^+$$

On mesure en spectrophotométrie (KONTRON) à 340 nm, la consommation de NADH en fonction du temps. On peut calculer ainsi une variation d'absorbance/minute (Δ Abs/minute) qui correspond à la vitesse initiale de la réaction enzymatique. Les résultats sont exprimés en activité spécifique (AS) soit en nmoles de NADH transformées/min/mg de protéines. Le taux de protéines totales est évalué par la méthode du BCA (Protein Assay Reagent-PIERCE LTD)

$$AS = 81{,}25 \times \Delta \text{ Abs/min} \times \frac{1}{\text{mg de protéines}}$$

3. Résultats

Mesure de l'activité glycéro-3-phosphate déshydrogénase ($G_3PDH$).

Les quantités de $NAD^+$ au bout de 8 jours en fonction des traitement des cultures sont reportées dans le tableau 3 ci-dessous.

Tableau 3

| Appréciation de la lipogénèse par mesure de la DO, exprimée en nmoles $NAD^+$, pour l'extrait de PB et pour la phytosphingosine PS | | | |
|---|---|---|---|
| PB (µg/mL) | PS (µg/mL) | D.O. (nmoles $NAD^+$) | |
| | | moyenne | écart |
| 0 (témoin) | 0 (témoin) | 0,0016 | 0,000195 |
| 25 | 0 | 0,0004 | 0,000015 |
| 0 | 0,5 | 0,0014 | 0,00009 |
| 0 | 1 | 0,00165 | 0,00006 |
| 25 | 1 | 0,00036 | 0,00002 |
| 25 | 0,5 | 0,00043 | 0,000048 |

Les résultats du tableau 3 ci-dessus sont également représentés à la figure 1.

Après 8 jours de contact avec les milieux conditionnés par des adipocytes matures, nous observons un ralentissement de la maturation des préadipocytes avec les milieux contenant du PB (inhibition de l'activité de la $G_3PDH$), ce qui correspond à une inhibition du recrutement des préadipocytes, dont l'activité de l'enzyme marqueur de maturation est inhibée de 75 %.

La phytosphingosine ne modifie pas ce profil anti-lipogénétique : l'association PS+PB provoque entre 74 et 79 % d'inhibition de l'activité de la $G_3PDH$, l'association PS 1 µg/mL + PB 25 µg/mL entraîne même un ralentissement de la lipogenèse de ces préadipocytes en cours de maturation légèrement supérieur au PB seul.

b) Analyse de la morphologie des adipocytes

Parallèlement, la morphologie des adipocytes a été analysée par observation directe des cellules dans les

boites de Pétri, sous microscope à phase inverse (Olympus BH2). Les cellules sont considérées comme différenciées par analyse morphologique lorsqu'elles acquièrent un contour rond et que leur cytoplasme est rempli de gouttelettes lipidiques. Inversement, une diminution de la quantité de vacuoles lipidiques associée à une forme plus allongée témoigne d'un ralentissement de cette maturation.

En présence de l'association PS+PB, les cellules sont caractérisées par une délipidation très marquée des adipocytes ce qui est en accord avec la baisse d'activité de l'enzyme $G_3$PDH précédemment mesurée.

Les figures 2, 3 et 4 montrent respectivement:

Figure 2: les cellules d'une culture témoin à J7. Dans cette culture, les cellules sont très peu différenciées, il y a peu de vacuoles lipidiques. C'est le début du traitement.
Figure 3: les cellules d'une culture témoin à J21. Les cellules se sont chargées en vacuoles lipidiques.
Figure 4: les cellules d'une culture traitée par l'association PB 25 μg/mL + PS 1 μg/mL

c) Conclusion

Cette étude montre que le traitement par l'association PB+PS d'adipocytes matures véhicule une information capable de ralentir globalement l'accumulation de triglycérides dans des préadipocytes. La baisse d'activité de l'enzyme lipogénétique $G_3$PDH conduit à une déplétion en gouttelettes lipidiques intracellulaires importantes.

L'adjonction de phytosphingosine capable de stimuler la sécrétion de leptine, n'a pas inhibé l'action massive du PB sur le déstockage de lipides.

Le maintien de leptine dans l'environnement proche des adipocytes pourrait ainsi exercer son rôle de molécule signal s'opposant à l'augmentation de la masse adipeuse. On est tenté de penser qu'un ensemble de cellules contenant des cellules vidées d'une partie de leur contenu en triglycérides par lipolyse en continuant à émettre un message leptinique en rétrocontrôle devrait contribuer à la réduction du stockage de graisse par le pannicule adipeux.

Exemple 3

Mise en évidence, sur des adipocytes humains en culture, de l'activité de la phytosphingosine et de l'association de la phytosphingosine avec un extrait de *Coleus forskohlii,* sur la production de la leptine et sur la lipolyse

Des adipocytes humains provenant d'une plastie d'une femme âgée de 41 ans, commercialisés par ZEN-BIO (USA), ont été utilisés à un stade avancé de la maturation adipocytaire. La réception de ces cellules en culture a eu lieu 16 jours après l'ensemencement. Ils sont cultivés dans un milieu spécifique assurant leur différenciation durant tout le traitement.

J -16: ensemencement

J 0: début du traitement par l'extrait de *Coleus* (PB) et/ou la phytosphingosine (PS)

J 6 à J 18 : dosages des différents paramètres.

Dans un premier temps, les propriétés du *Coleus* décrites précédemment pour les adipocytes murins 3T3F442A ont été vérifiées sur ces cellules humaines : activité lipolytique par mesure de la libération de glycérol et d'acides gras non estérifiés.

Le tableau 4 ci-dessous indique les valeurs de libération de glycérol dans les cultures témoin et traitées par la PS.

Tableau 4

| Libération de glycérol, exprimée en μg/mL, par les adipocytes humains en culture, traités ou non par 25μg/mL de phytosphingosine, en fonction de la durée de culture | | |
|---|---|---|
| Jours | glycérol (pg/mL) | |
| | Témoin (PS = 0 μg/mL) | PS = 25 μg/mL |
| 3 | 73, 78 | 174,4 |
| 6 | 120,8 | 196,2 |
| 9 | 147,2 | 302,9 |
| 12 | 198,4 | 416,4 |

(suite)

| Libération de glycérol, exprimée en μg/mL, par les adipocytes humains en culture, traités ou non par 25μg/mL de phytosphingosine, en fonction de la durée de culture | | |
|---|---|---|
| Jours | glycérol (pg/mL) | |
| | Témoin (PS = 0 μg/mL) | PS = 25 μg/mL |
| 15 | 196,3 | 460,6 |
| 18 | 230,9 | 659,8 |
| 21 | 276,6 | 777,1 |
| 23 | 238,9 | 687 |

Il apparaît très clairement que le *Coleus* stimule très fortement la lipolyse adipocytaire (+128 % d'augmentation de libération de glycérol à 18 jours par rapport au témoin)

Par une mesure de libération d'acides gras non estérifiés, on observe de plus que le *Coleus* provoque une forte hydrolyse des triglycérides adipocytaires en libérant parallèlement au glycérol une grande quantité d'acides gras non estérifiés à la dose 25 μg/mL.

D'autres essais montrent que la phytosphingosine induit chez les adipocytes humains comme chez les adipocytes murins une augmentation de la sécrétion de leptine, les doses les plus efficaces étant inférieures pour l'adipocyte humain.

Parallèlement à la libération de leptine, la phytosphingosine à la dose de 6 ng/mL est responsable d'une libération dans le temps de glycérol sans libération concomitante d'acides gras non estérifiés. Cette observation pourrait correspondre à une nouvelle forme de lipolyse propre à la leptine, déjà décrite dans des publications.

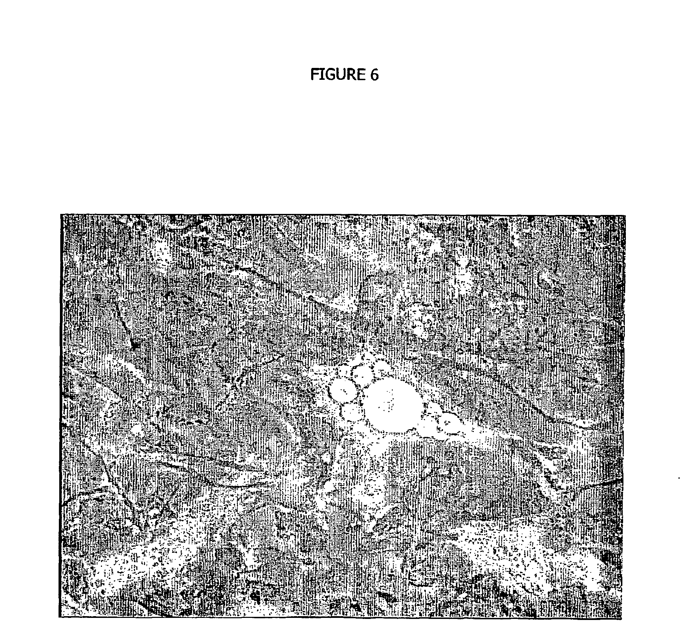

Les figures 5 et 6 présentent l'évolution de la morphologie des cellules dans les conditions qui viennent d'être évoquées.

La figure 5 présente une culture témoin d'adipocytes humains au jour J18.

On y voit nettement une importante quantité de vacuoles grasses.

La figure 6 présente une culture d'adipocytes humains au jour J18, après traitement par l'association de *Coleus* (25 μg/mL) avec la phytosphingosine (6ng/mL). Il apparaît clairement que l'association phytosphingosine - *Coleus* entraîne un déstockage massif et visible, avec des gouttelettes lipidiques moins nombreuses et de taille inférieure. Parallèlement, un plus grand nombre de cellules reprennent une apparence de cellules peu matures (forme allongée et absence de vacuoles lipidiques).

Ainsi, l'association de ces deux actifs conduit à une puissante réduction du stock graisseux, la modulation de leptine dans l'environnement proche de l'adipocyte humain étant une étape clé de cette action.

<u>Exemple 4</u>

Formule hydro/alcoolique amincissante.

| | |
|---|---|
| Eau | qsp 100 % |
| Alcool dénaturé | 42 % |
| PPG-3 myristyl éther | 10 % |
| Parfum | 0,20 % |
| Phytosphingosine | 0,10% |

<u>Exemple 5</u>

Emulsion fluide amincissante.

| | |
|---|---|
| PPG-2 isoceteth-20 acétate | 2 % |
| Poloxamer 407 | 0,50 % |
| Propylène glycol isoceteth-3 acétate | 15 % |
| Pentacyclométhicone | 15 % |
| Eau | qsp.100 % |

(suite)

| | |
|---|---|
| Butylène glycol | 3 % |
| Conservateurs | q.s. |
| Extrait de *Coleus forskohlii* (à 80 % de forskoline) | 0,1% |
| Gomme xanthane | 0,05% |
| Acrylates/c10-30 alkyl acrylate crosspolymer | 0,04% |

| | |
|---|---|
| Neutralisant | q.s. |
| Polyacrylamide c13-14 isoparaffin laureth-7 | 0,50% |
| Parfum | 0,20% |
| Phytosphingosine | 0,05% |

Exemple 6

Crème amincissante.

| | |
|---|---|
| Steareth-2 | 0,50% |
| Steareth-21 | 1,75% |
| Alcool cétylique | 0,30% |
| Alcool stéarylique | 0,30% |
| Acide stéarique | 0,50% |
| Stearate éthyle-2-hexyle | 4,00% |
| Cetearyl isononanoate | 3,00% |
| Squalane | 4,00% |
| IBMX | 1,00% |
| Diméthicone | 0,40% |
| Eau qsp.100.00% | |
| Glycérine | 2,00% |
| Butylène glycol | 3,00% |
| Conservateurs | q.s. |
| Acrylates/c10-30 alkyl acrylate crosspolymer | 0,35% |
| Gomme xanthane | 0,10% |
| Sodium hyaluronate | 0,02% |
| Neutralisant | q.s. |
| Polyacrylamide c13-14 isoparaffin laureth 7 | 0,50% |
| Alcool dénaturé | 5,00% |
| Parfum | 0,20% |
| Phytosphingosine | 0,002% |

**Revendications**

1. Utilisation cosmétique de la phytosphingosine ou de l'un de ses sels cosmétiquement acceptables , en particulier son chlorhydrate, en tant qu'agent amincissant, ledit agent amincissant étant contenu dans une composition cosmétique.

2. Utilisation cosmétique de la phytosphingosine ou de l'un de ses sels cosmétiquement acceptables, en particulier son chlorhydrate, comme agent actif stimulant la synthèse de la leptine par les adipocytes, ledit agent amincissant étant contenu dans une composition cosmétique.

3. Utilisation cosmétique selon la revendication 1 ou 2, **caractérisée en ce que** la phytosphingosine ou l'un de ses sels, en particulier son chlorhydrate, est utilisé à une concentration comprise entre 0,001% et 1%, et de préférence

entre 0,05% et 0,5% en poids par rapport au poids total de ladite composition.

4. Utilisation selon l'une des revendications 1 à 3, **caractérisée en ce que** la phytosphingosine ou son sel cosmétiquement acceptable, en particulier son chlorhydrate, est en combinaison dans ladite composition avec au moins un agent lipolytique cosmétiquement acceptable.

5. Utilisation selon la revendication 4, **caractérisé en ce que** ledit agent lipolytique est l'adénosine-3',5'-cyclique monophosphate (AMPc) ou l'un de ses dérivés cosmétiquement acceptables, choisi de préférence parmi ses sels et ses dérivés acylés, notamment un dérivé mono- ou dibutyryle.

6. Utilisation selon la revendication 5, **caractérisée en ce** l'AMPc ou son dérivé est utilisé à une concentration comprise entre 0,001 % et 5 % en poids par rapport au poids total de ladite composition.

7. Utilisation selon la revendication 4, **caractérisée en ce que** ledit agent lipolytique est un agent activateur de l'enzyme adénylate cyclase.

8. Utilisation selon la revendication 7, **caractérisée en ce que** ledit agent activateur de l'enzyme adénylate cyclase est la forskoline ou un extrait de plante en contenant.

9. Utilisation selon la revendication 8, **caractérisée en ce que** la forskoline ou l'extrait de plante en contenant est utilisé à une concentration comprise entre 0,001% et 1%, de préférence entre 0,05% et 0,25%, en poids par rapport au poids total de ladite composition.

10. Utilisation selon la revendication 8 ou 9, **caractérisée en ce que** ledit extrait contenant de la forskoline est un extrait de *Coleus forskohlii* ou *Plectranthus barbatus*

11. Utilisation selon la revendication 7, **caractérisée en ce que** l'agent activateur de l'adénylate cyclase est un extrait de la plante *Tephrosia purpurea,* à une concentration comprise entre 0,001 % et 5 % en poids, de préférence entre 0,01 % et 5 % en poids par rapport au poids total de ladite composition.

12. Utilisation selon la revendication 4, **caractérisée en ce que** ledit agent lipolytique est un agent inhibiteur de l'enzyme phosphodiestérase.

13. Utilisation selon la revendication 12, **caractérisée en ce que** ledit agent inhibiteur de la phosphodiestérase est une xanthine, en particulier la 3-isobutyl-1-méthyl-xanthine ou IBMX, la caféine ou la théophylline.

14. Utilisation selon la revendication 13, **caractérisée en ce que** ladite xanthine est utilisée à une concentration comprise entre 0,001 % et 10 % en poids, de préférence comprise entre 0,01 % et 1 % en poids par rapport au poids de ladite composition.

15. Procédé de traitement cosmétique destiné à obtenir un effet amincissant du corps humain, **caractérisé en ce qu'**il consiste à appliquer sur les parties du corps où ledit effet est recherché une quantité efficace d'une composition cosmétique contenant de la phytosphingosine ou l'un de ses sels cosmétiquement acceptables, en particulier son chlorhydrate.

16. Procédé de traitement cosmétique selon la revendication 15, **caractérisé en ce que** ladite composition cosmétique contient en outre au moins un agent lipolytique cosmétiquement acceptable.

17. Procédé de traitement cosmétique selon la revendication 16, **caractérisé en ce que** l'agent lipolytique est choisi dans le groupe constitué par l'AMPc et ses dérivés, les activateurs d'adénylate cyclase, en particulier les extraits de *Tephrosia purpurea,* la forskoline et les extraits de plante en contenant tels que les extraits de *Coleus forskohlii* ou *Plectranthus barbatus,* et les Inhibiteurs de la phosphodiestérase, en particulier les xanthines, notamment la 3-isobutyl-1-méthyi-xanthine ou IBMX, la caféine et la théophylline, ledit agent lipolytique étant présent dans ladite composition à une concentration telle que définie dans les revendications 6, 9 et 14.

18. Composition cosmétique, notamment destinée à réduire les surcharges graisseuses sous-cutanées, **caractérisée en ce qu'**elle contient, à titre d'agents actifs,

- de la phytosphingosine ou l'un de ses sels cosmétiquement acceptables, en particulier son chlorhydrate, et
- au moins un agent lipolytique choisi dans le groupe constitué par l'AMPc et ses dérivés lipolytiques cosmétiquement acceptables, les agents activateurs de l'enzyme adénylate cyclase et les agents inhibiteurs de l'enzyme phosphodiestérase,

dans un véhicule cosmétiquement acceptable.

**19.** Composition cosmétique selon la revendication 18, **caractérisée en ce qu'**elle contient de 0,001 à 1 %, et de préférence de 0,05 à 0,5 %, en poids de phytosphingosine ou de l'un de ses sels cosmétiquement acceptables, en particulier son chlorhydrate.

**20.** Composition cosmétique selon la revendication 18 ou 19, **caractérisée en ce que** l'agent lipolytique est l'AMPc ou l'un de ses dérivés cosmétiquement acceptables, choisi de préférence parmi ses sels et ses dérivés acylés, notamment un dérivé mono- ou dibutyryle.

**21.** Composition cosmétique selon la revendication 20, **caractérisée en ce que** l'AMPc ou son dérivé lipolytique est présent à une concentration comprise entre 0,001 % et 5 % en poids par rapport au poids total de ladite composition.

**22.** Composition cosmétique selon la revendication 18, **caractérisée en ce que** l'agent lipolytique est un agent activateur de l'enzyme adénylate cyclase.

**23.** Composition cosmétique selon la revendication 22, **caractérisée en ce que** ledit agent activateur de l'enzyme adénylate cyclase est la forskoline ou un extrait de plante en contenant.

**24.** Composition cosmétique selon la revendication 23, **caractérisée en ce que** la forskoline ou l'extrait en contenant est présent à une concentration comprise entre 0,001% et 1%, et de préférence entre 0,05% et 0,25% en poids par rapport au poids total de ladite composition.

**25.** Composition cosmétique selon la revendication 23 ou 24, **caractérisée en ce que** l'extrait de plante est un extrait de *Coleus forskohlii* ou *Plectranthus barbatus*

**26.** Composition cosmétique selon la revendication 22, **caractérisée en ce que** ledit agent activateur de l'adénylate cyclase est un extrait de la plante *Tephrosia purpurea.*

**27.** Composition cosmétique selon la revendication 26, **caractérisée en ce que** l'extrait de *Tephrosia purpurea* est présent à une concentration comprise entre 0,001 % et 5 % en poids, de préférence entre 0,01 % et 5 % en poids par rapport au poids total de la composition.

**28.** Composition cosmétique selon la revendication 18, **caractérisée en ce que** l'agent lipolytique est un agent inhibiteur de l'enzyme phosphodiestérase.

**29.** Composition cosmétique selon la revendication 28, **caractérisée en ce que** ledit agent inhibiteur de la phosphodiestérase est une xanthine, en particulier la 3-isobutyl-1-méthyi-xanthine ou IBMX, la caféine ou la théophylline.

**30.** Composition cosmétique selon la revendication 29, **caractérisée en ce que** ladite xanthine est présente à une concentration comprise entre 0,001 % et 10%, de préférence comprise entre 0,01 % et 1 %, en poids par rapport au poids total de la composition.

## Claims

**1.** Cosmetic use of phytosphingosine or of one of its cosmetically acceptable salts, particularly its hydrochloride, as a slimming agent, said slimming agent being contained in a cosmetic composition.

**2.** Cosmetic use of phytosphingosine or of one of its cosmetically acceptable salts, particularly its hydrochloride, as an active agent which stimulates the synthesis of leptin by adipocytes, said slimming agent being contained in a cosmetic composition.

3. The cosmetic use according to claim 1 or 2, **characterised in that** the phytosphingosine or one of its salts, particularly its hydrochloride, is used at a concentration of between 0.001% and 1%, and preferably between 0.05% and 0.5%, by weight with respect to the total weight of said composition.

4. The use according to one of claims 1 to 3, **characterised in that** the phytosphingosine or its cosmetically acceptable salt, particularly its hydrochloride, is in combination in said composition with at least one cosmetically acceptable lipolytic agent.

5. The use according to claim 4, **characterised in that** said lipolytic agent is adenosine-3',5'-cyclic monophosphate (cAMP) or one of its cosmetically acceptable derivatives, preferably selected from its salts and its acylated derivatives, notably a mono- or dibutyryl derivative.

6. The use according to claim 5, **characterised in that** the cAMP or its derivative is used at a concentration of between 0.001% and 5% by weight with respect to the total weight of said composition.

7. The use according to claim 4, **characterised in that** said lipolytic agent is an adenylate cyclase enzyme activating agent.

8. The use according to claim 7, **characterised in that** said adenylate cyclase enzyme activating agent is forskolin or a plant extract containing it.

9. The use according to claim 8, **characterised in that** the forskolin or the plant extract containing it is used at a concentration of between 0.001% and 1%, preferably between 0.05% and 0.25%, by weight with respect to the total weight of said composition.

10. The use according to claim 8 or 9, **characterised in that** said extract containing forskolin is an extract of *Coleus forskohlii* or *Plectranthus barbatus*.

11. The use according to claim 7, **characterised in that** the adenylate cyclase activating agent is an extract of the plant *Tephrosia purpurea,* at a concentration of between 0.001% and 5% by weight, preferably between 0.01% and 5% by weight, with respect to the total weight of said composition.

12. The use according to claim 4, **characterised in that** said lipolytic agent is a phosphodiesterase enzyme inhibiting agent.

13. The use according to claim 12, **characterised in that** said phosphodiesterase inhibiting agent is a xanthine, particularly 3-isobutyl-1-methyl-xanthine or IBMX, caffeine or theophylline.

14. The use according to claim 13, **characterised in that** said xanthine is used at a concentration of between 0.001% and 10% by weight, preferably of between 0.01% and 1% by weight, with respect to the weight of said composition.

15. A method of cosmetic treatment intended for obtaining a slimming effect on the human body, **characterised in that** it consists in applying, on the parts of the body where said effect is sought, an effective amount of a cosmetic composition containing phytosphingosine or one of its cosmetically acceptable salts, particularly its hydrochloride.

16. The method of cosmetic treatment according to claim 15, **characterised in that** said cosmetic composition further contains at least one cosmetically acceptable lipolytic agent.

17. The method of cosmetic treatment according to claim 16, **characterised in that** the lipolytic agent is selected from the group consisting of cAMP and its derivatives, activators of adenylate cyclase, particularly extracts of *Tephrosia purpurea,* forskolin and plant extracts containing it such as extracts of *Coleus forskohlii* or *Plectranthus barbatus,* and inhibitors of phosphodiesterase, particularly xanthines, notably 3-isobutyl-1-methyl-xanthine or IBMX, caffeine and theophylline, said lipolytic agent being present in said composition at a concentration as defined in claims 6, 9 and 14.

18. A cosmetic composition, notably intended for reducing subcutaneous excess fat, **characterised in that** it contains, as active agents,

- phytosphingosine or one of its cosmetically acceptable salts, particularly its hydrochloride, and
- at least one lipolytic agent selected from the group consisting of cAMP and its cosmetically acceptable lipolytic derivatives, adenylate cyclase enzyme activating agents and phosphodiesterase enzyme inhibiting agents,

in a cosmetically acceptable vehicle.

**19.** The cosmetic composition according to claim 18, **characterised in that** it contains from 0.001 to 1%, and preferably from 0.05 to 0.5%, by weight of phytosphingosine or of one of its cosmetically acceptable salts, particularly its hydrochloride.

**20.** The cosmetic composition according to claim 18 or 19, **characterised in that** the lipolytic agent is cAMP or one of its cosmetically acceptable derivatives, preferably selected from its salts and its acylated derivatives, notably a mono- or dibutyryl derivative.

**21.** The cosmetic composition according to claim 20, **characterised in that** cAMP or its lipolytic derivative is present at a concentration of between 0.001% and 5% by weight with respect to the total weight of said composition.

**22.** The cosmetic composition according to claim 18, **characterised in that** the lipolytic agent is an adenylate cyclase enzyme activating agent.

**23.** The cosmetic composition according to claim 22,
**characterised in that** said adenylate cyclase enzyme activating agent is forskolin or a plant extract containing it.

**24.** The cosmetic composition according to claim 23, **characterised in that** the forskolin or the extract containing it is present at a concentration of between 0.001% and 1%, and preferably between 0.05% and 0.25%, by weight with respect to the total weight of said composition.

**25.** The cosmetic composition according to claim 23 or 24, **characterised in that** the plant extract is an extract of *Coleus forskohlii* or *Plectranthus barbatus*

**26.** The cosmetic composition according to claim 22,
**characterised in that** said adenylate cyclase activating agent is an extract of the plant *Tephrosia purpurea.*

**27.** The cosmetic composition according to claim 26, **characterised in that** the extract de *Tephrosia purpurea* is present at a concentration of between 0.001% and 5% by weight, preferably between 0.01% and 5% by weight, with respect to the total weight of the composition.

**28.** The cosmetic composition according to claim 18, **characterised in that** the lipolytic agent is a phosphodiesterase enzyme inhibiting agent.

**29.** The cosmetic composition according to claim 28, **characterised in that** said phosphodiesterase inhibiting agent is a xanthine, particularly 3-isobutyl-1-methyl-xanthine or IBMX, caffeine or theophylline.

**30.** The cosmetic composition according to claim 29, **characterised in that** said xanthine is present at a concentration of between 0.001% and 10%, preferably of between 0.01% and 1%, by weight with respect to the total weight of the composition.

**Patentansprüche**

**1.** Kosmetische Verwendung von Phytosphingosin oder von einem seiner kosmetisch akzeptablen Salze, insbesondere seines Chlorhydrats, als Schlankmacher, wobei der Schlankmacher in einer kosmetischen Zusammensetzung enthalten ist.

**2.** Kosmetische Verwendung von Phytosphingosin oder von einem seiner kosmetisch akzeptablen Salze, insbesondere seines Chlorhydrats, als Wirkstoff, welcher die Synthese von Leptin durch Adipozyten stimuliert, wobei der genannte Wirkstoff in einer kosmetischen Zusammensetzung enthalten ist.

**3.** Kosmetische Zusammensetzung nach Anspruch 1 oder 2, **dadurch gekennzeichnet, daß** das Phytosphingosin oder eines seiner Salze, insbesondere sein Chlorhydrat, in einer Konzentration zwischen 0,001 und 1 Gew.-% und vorzugsweise zwischen 0,05 und 0,5 Gew.-% bezogen auf das Gesamtgewicht der Zusammensetzung verwendet wird.

**4.** Verwendung nach einem der Ansprüche 1 bis 3, **dadurch gekennzeichnet, daß** das Phytosphingosin oder dessen kosmetisch akzeptables Salz, insbesondere dessen Chlorhydrat, in der genannten Zusammensetzung mit wenigstens einem kosmetisch akzeptablen lipolytischen Mittel in Kombination vorliegt.

**5.** Verwendung nach Anspruch 4, **dadurch gekennzeichnet, daß** das lipolytische Mittel zyklisches Adenosin-3',5'-monophosphat (cAMP) oder eines seiner kosmetisch akzeptablen Derivate, vorzugsweise ausgewählt aus seinen Salzen und seinen Acylderivaten, insbesondere ein Mono- oder Dibutyryl-Derivat ist.

**6.** Verwendung nach Anspruch 5, **dadurch gekennzeichnet, daß** das cAMP oder dessen Derivat in einer Konzentration zwischen 0,001 und 5 Gew.-% bezogen auf das Gesamtgewicht der Zusammensetzung verwendet wird.

**7.** Verwendung nach Anspruch 4, **dadurch gekennzeichnet, daß** das lipolytische Mittel ein Aktivator des Enzyms Adenylatzyklase ist.

**8.** Verwendung nach Anspruch 7, **dadurch gekennzeichnet, daß** der genannte Aktivator des Enzyms Adenylatzyklase Forskolin oder ein dieses enthaltender Pflanzenextrakt ist.

**9.** Verwendung nach Anspruch 8, **dadurch gekennzeichnet, daß** das Forskolin oder der dieses enthaltende Pflanzenextrakt in einer Konzentration zwischen 0,001 und 1 Gew.-%, vorzugsweise zwischen 0,05 und 0,25 Gew.-% bezogen auf das Gesamtgewicht der Zusammensetzung verwendet wird.

**10.** Verwendung nach Anspruch 8 oder 9, **dadurch gekennzeichnet, daß** der Forskolin enthaltende Extrakt ein Coleus forskohlii- oder ein Plectranthus barbatus-Extrakt ist.

**11.** Verwendung nach Anspruch 7, **dadurch gekennzeichnet, daß** der Aktivator der Adenylatzyklase ein Extrakt der Pflanze Tephrosia purpurea in einer Konzentration zwischen 0,001 und 5 Gew.-%, vorzugsweise zwischen 0,01 und 5 Gew.-% bezogen auf das Gesamtgewicht der Zusammensetzung ist.

**12.** Verwendung nach Anspruch 4, **dadurch gekennzeichnet, daß** das lipolytische Mittel ein das Enzym Phosphodiesterase hemmendes Mittel ist.

**13.** Verwendung nach Anspruch 12, **dadurch gekennzeichnet, daß** das die Phosphodiesterase hemmende Mittel ein Xanthin, insbesondere 3-Isobutyl-1-Methyl-Xanthin oder IBMX, Coffein oder Theophyllin ist.

**14.** Verwendung nach Anspruch 13, **dadurch gekennzeichnet, daß** das Xanthin in einer Konzentration zwischen 0,001 und 10 Gew-%, vorzugsweise zwischen 0,01 und 1 Gew.-% bezogen auf das Gewicht der Zusammensetzung verwendet wird.

**15.** Verfahren zur kosmetischen Behandlung, welches dazu bestimmt ist, eine schlankmachende Wirkung des menschlichen Körpers zu erzielen, **dadurch gekennzeichnet, daß** es darauf beruht, auf die Teile des Körpers, bei denen die genannte Wirkung gewünscht wird, eine wirkungsvolle Menge einer kosmetischen Zusammensetzung, welche Phytosphingosin oder eines seiner kosmetisch akzeptablen Salze, insbesondere dessen Chlorhydrat enthält, aufzutragen.

**16.** Verfahren zur kosmetischen Behandlung nach Anspruch 15, **dadurch gekennzeichnet, daß** die kosmetische Zusammensetzung ferner wenigstens ein kosmetisch akzeptables lipolytisches Mittel enthält.

**17.** Verfahren zur kosmetischen Behandlung nach Anspruch 16, **dadurch gekennzeichnet, daß** das lipolytische Mittel aus der Gruppe bestehend aus cAMP und dessen Derivaten, den Aktivatoren von Adenylatzyklase, insbesondere den Tephrosia pupruea-Extrakten, dem Forskolin und den dieses enthaltenden Pflanzenextrakten, wie den Coleus forskohlü- oder Plectranthus barbatus-Extrakten, sowie den Inhibitoren der Phosphodiesterase, insbesondere den Xanthinen, vor allem 3-Isobutyl-1-Methyl-Xanthin oder IBMX, Coffein und Theophyllin ausgewählt ist, wobei das lipolytische Mittel in der Zusammensetzung in einer Konzentration vorliegt, wie sie in den Ansprüchen 6, 9 und 14

definiert ist.

**18.** Kosmetische Zusammensetzung, die insbesondere dazu bestimmt ist, die subkutane Fettüberlastung zu verringern, **dadurch gekennzeichnet, daß** sie als Wirkstoffe

- Phytosphingosin oder eines seiner kosmetisch akzeptablen Salze, insbesondere dessen Chlorhydrat, und
- wenigstens ein lipolytisches Mittel, welches aus der Gruppe bestehend aus cAMP und seinen kosmetisch akzeptablen lipolytischen Derivaten, den Aktivatoren des Enzyms Adenylatzkylase und das Enzym Phosphodiesterase hemmenden Mitteln ausgewählt ist,

in einem kosmetisch akzeptablen Vehikel enthält.

**19.** Kosmetische Zusammensetzung nach Anspruch 18, **dadurch gekennzeichnet, daß** sie 0,001 bis 1 Gew.-% und vorzugsweise 0,05 bis 0,5 Gew.-% Phytosphingosin oder von einem seiner kosmetisch akzeptablen Salze, insbesondere dessen Chlorhydrat enthält.

**20.** Kosmetische Zusammensetzung nach Anspruch 18 oder 19, **dadurch gekennzeichnet, daß** das lipolytische Mittel cAMP oder eines seiner kosmetisch akzeptablen Derivate, vorzugsweise ausgewählt aus dessen Salzen oder dessen Acylderivaten, insbesondere ein Mono- oder Dibutyryl-Derivat ist.

**21.** Kosmetische Zusammensetzung nach Anspruch 20, **dadurch gekennzeichnet, daß** das cAMP oder dessen lipolytisches Derivat in einer Konzentration zwischen 0,001 und 5 Gew.-% bezogen auf das Gesamtgewicht der Zusammensetzung vorliegt.

**22.** Kosmetische Zusammensetzung nach Anspruch 18, **dadurch gekennzeichnet, daß** das lipolytische Mittel ein Aktivator des Enzyms Adenylatzyklase ist.

**23.** Kosmetische Zusammensetzung nach Anspruch 22, **dadurch gekennzeichnet, daß** der Aktivator des Enzyms Adenylatzyklase Forskolin oder ein dieses enthaltender Pflanzenextrakt ist.

**24.** Kosmetische Zusammensetzung nach Anspruch 23, **dadurch gekennzeichnet, daß** das Forskolin oder der dieses enthaltende Extrakt in einer Konzentration zwischen 0,001 und 1 Gew.-% und vorzugsweise zwischen 0,05 und 0,25 Gew.-% bezogen auf das Gesamtgewicht der Zusammensetzung vorliegt.

**25.** Kosmetische Zusammensetzung nach Anspruch 23 oder 24, **dadurch gekennzeichnet, daß** der Pflanzenextrakt ein Coleus forskohlii- oder ein Plectranthus barbatus-Extrakt ist.

**26.** Kosmetische Zusammensetzung nach Anspruch 22, **dadurch gekennzeichnet, daß** der Aktivator der Adenylatzyklase ein Extrakt der Pflanze Tephrosia purpurea ist.

**27.** Kosmetische Zusammensetzung nach Anspruch 26, **dadurch gekennzeichnet, daß** der Extrakt aus Tephrosia purpurea in einer Konzentration zwischen 0,001 und 5 Gew.-%, vorzugsweise zwischen 0,01 und 5 Gew.-% bezogen auf das Gesamtgewicht der Zusammensetzung vorliegt.

**28.** Kosmetische Zusammensetzung nach Anspruch 18, **dadurch gekennzeichnet, daß** das lipolytische Mittel ein das Enzym Phosphodiesterase hemmendes Mittel ist.

**29.** Kosmetische Zusammensetzung nach Anspruch 28, **dadurch gekennzeichnet, daß** das die Phosphodiesterase hemmende Mittel ein Xanthin, insbesondere 3-Isobutyl-1-Methyl-Xanthin oder IBMX, Coffein oder Theophyllin ist.

**30.** Kosmetische Zusammensetzung nach Anspruch 29, **dadurch gekennzeichnet, daß** das Xanthin in einer Konzentration zwischen 0,001 und 10 Gew.-%, vorzugsweise zwischen 0,01 und 1 Gew.-% bezogen auf das Gesamtgewicht der Zusammensetzung vorliegt.

FIGURE 1

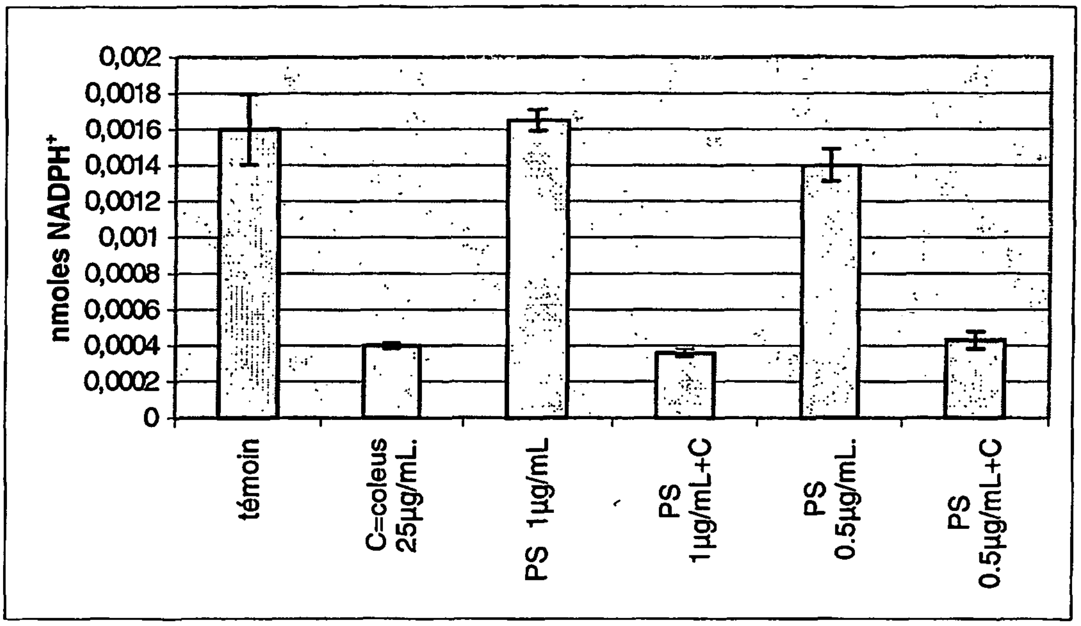

0,002
0,0018
0,0016
0,0014
0,0012
0,001
0,0008
0,0006
0,0004
0,0002
0
nmoles NADPH+
témoin
C=coleus 25µg/mL.
PS 1µg/mL
PS 1µg/mL+C
PS 0.5µg/mL.
PS 0.5µg/mL+C

FIGURE 2

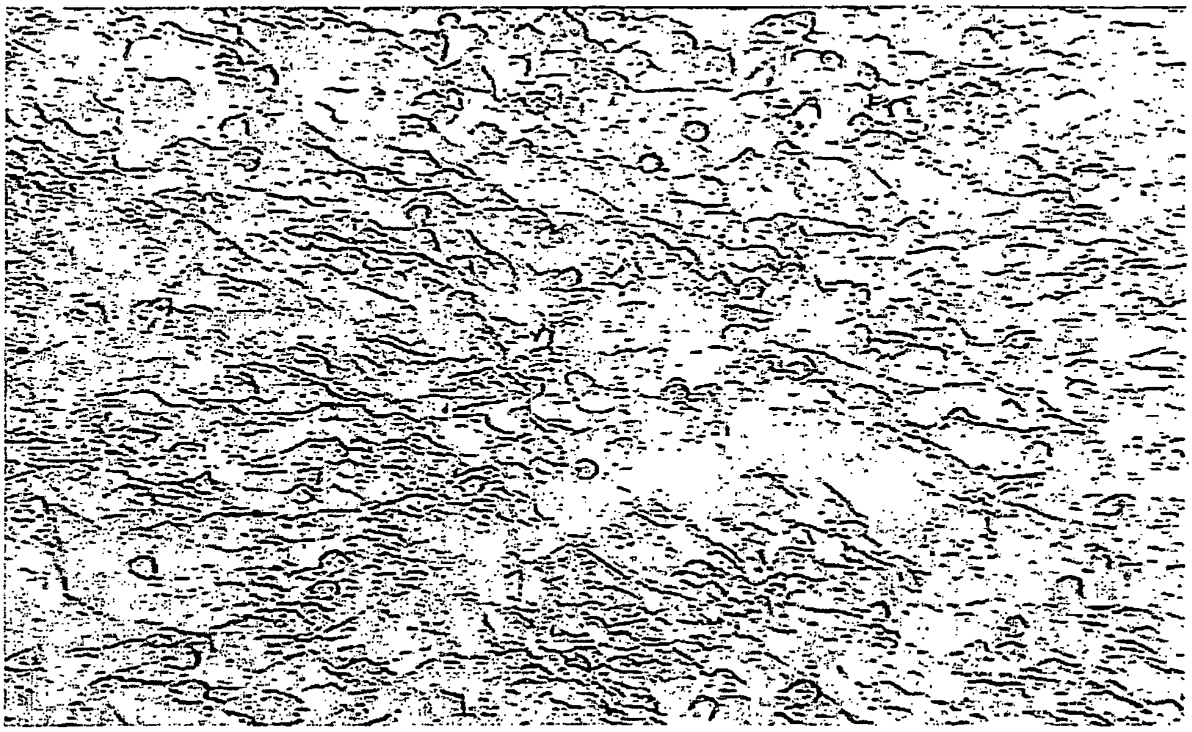

FIGURE 3

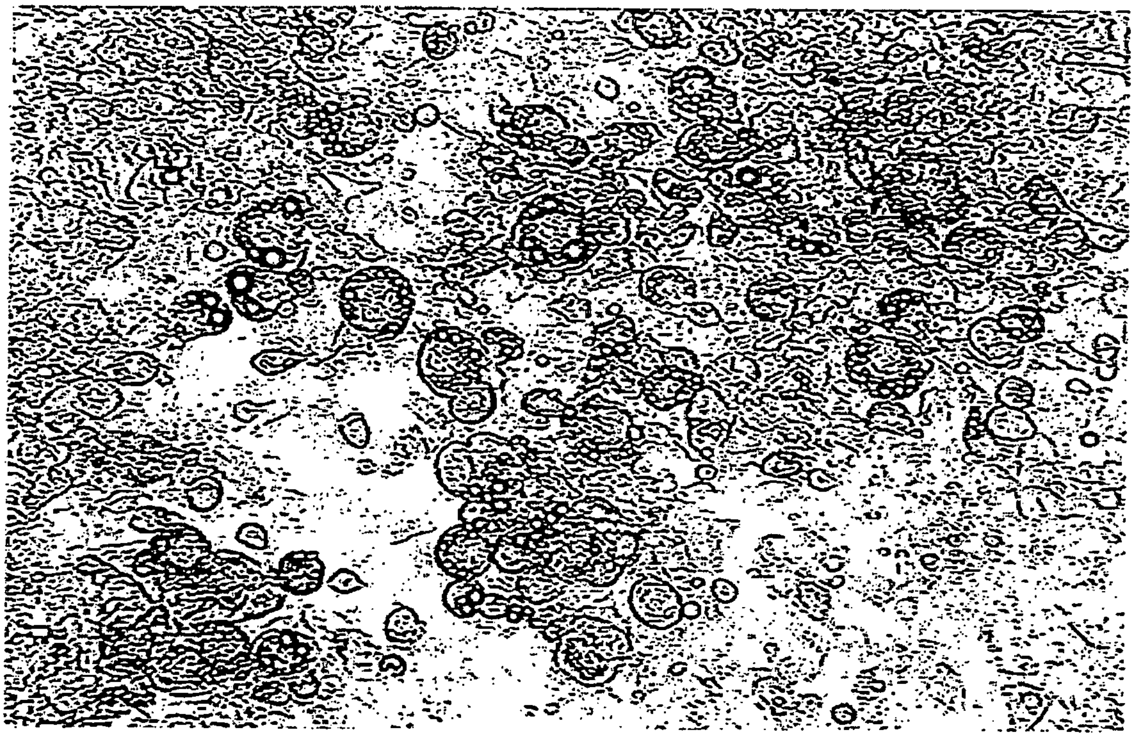

FIGURE 4

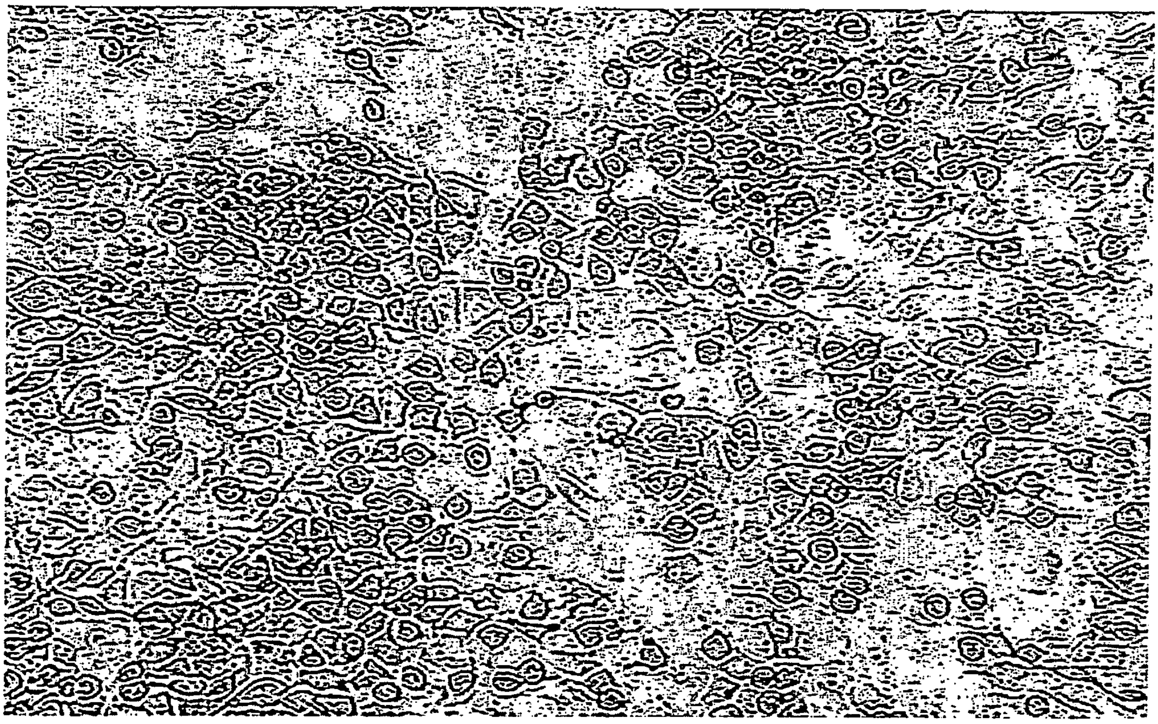

FIGURE 5

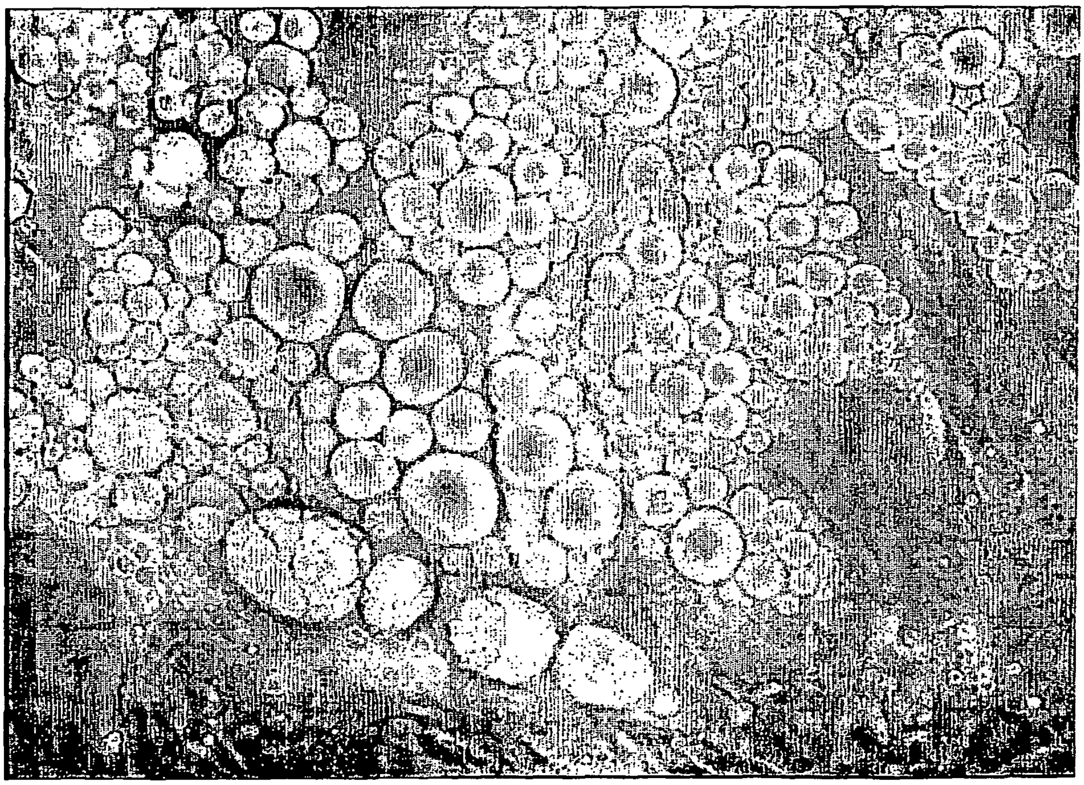

FIGURE 6